# EUROPÄISCHE PATENTSCHRIFT

(11) **EP 3 996 618 B1**
(45) Veröffentlichungstag und Bekanntmachung des Hinweises auf die Patenterteilung: **26.06.2024**
(21) Anmeldenummer: 20751473.8
(22) Anmeldetag: 13.07.2020
(51) Int. Cl.: A61B 18/12, A61F 7/00, A61B 18/00, A61B 18/14

(54) **KONTRAZEPTIONSVORRICHTUNG**
CONTRACEPTION DEVICE
DISPOSITIF DE CONTRACEPTION

(30) Priorität: 12.07.2019 DE 102019119026
(43) Veröffentlichungstag der Anmeldung: 18.05.2022
(73) Patentinhaber: Grohs, Niklas, 21493 Schwarzenbeck (DE)
(72) Erfinder: Grohs, Niklas, 21493 Schwarzenbeck (DE)
(74) Vertreter: Habermann Intellectual Property Partnerschaft von Patentanwälten mbB
(86) Internationale Anmeldenummer: PCT/EP2020/069774
(87) Internationale Veröffentlichungsnummer: WO 2021/009128

(56) Entgegenhaltungen:
- EP-A1- 2 810 694
- DE-U1-202014 004 985
- US-A- 1 633 878
- US-A- 5 063 939

## Beschreibung

Die Erfindung betrifft eine Kontrazeptionsvorrichtung zur Reduzierung einer Qualität von Spermien eines Säugetiers mit Hodenabstieg, durch ein lokales Erwärmen zumindest eines Nebenhodens des Säugetiers. Die Erfindung ist in den beigefügten Ansprüchen definiert. Hier offenbarte Aspekte, Ausführungsformen und Beispiele, die nicht in den Umfang der beigefügten Ansprüche fallen, sind nicht Teil der Erfindung und werden lediglich zu Veranschaulichungszwecken bereitgestellt.

Im Laufe der Menschheitsgeschichte sind unterschiedlichste Verfahren und Vorrichtungen zur Geburtenkontrolle und Empfängnisverhütung entwickelt worden, die zunächst bei Nutz-und Haustierpopulationen angewendet wurden. Mit fortschreitender Entwicklung der Medizin sind darüber hinaus auch Verfahren, Vorrichtungen und Medikamente entwickelt worden, die es Menschen ermöglichen, ihre Fähigkeit zur Zeugung von Kindern oder ihre Möglichkeit zur Empfängnis zu steuern. Ziel der bekannten Verfahren zur Empfängnisverhütung ist stets, zu verhindern, dass ein fruchtbares Spermium mit einer befruchtungsfähigen Eizelle verschmilzt und/ oder, dass sich die befruchtete Eizelle in die Gebärmutterschleimhaut einnistet. Besonders weit verbreitete Verfahren, welche die Zeugung von Nachkommen nicht dauerhaft ausschließen, sind beispielsweise die Verwendung von Kondomen und Diaphragmen oder die Einnahme von Hormonen, etwa in Form der sogenannten Antibabypille. Außerdem sind Verfahren bekannt, welche die Fähigkeit zur Zeugung von Kindern oder die Möglichkeit zur Empfängnis dauerhaft einschränken, beispielsweise die Sterilisation.

Darüber hinaus sind auch Verfahren bekannt, bei denen die Spermien noch in den Nebenhoden durch Erwärmen derart verändert werden, dass ihre Fähigkeit zur Befruchtung einer Eizelle wesentlich beeinträchtigt ist. Durch derartige Wärmebehandlungsverfahren wird üblicherweise eine Qualität der Spermien reduziert, insbesondere die Motilität und die Morphologie, aber auch die Spermiengesamtzahl im Ejakulat. Beispielsweise kann sich eine Veränderung der Morphologie des Spermienschwanzes auf die Motilität, insbesondere die Vorwärtsbeweglichkeit auswirken, sodass das Spermium die Eizelle nicht erreichen kann.

Die typische Temperatur im Hoden beträgt beim Menschen mit etwa 34°C bis 35°C etwas weniger als die typische Körpertemperatur. Aus der DE 100 27 378 B4 ist eine Vorrichtung bekannt, mit der die Hoden dauerhaft in den Leistenkanälen fixierbar sind. Dadurch können die Hoden über längere Zeiträume hinweg auf Körpertemperatur gehalten werden. Durch diese Erwärmung wird eine Einschränkung der Zeugungsfähigkeit des Mannes erreicht.

Weiterhin ist bekannt, dass durch mehrmaliges Baden der Hoden in 45°C heißem Wasser die Qualität der Spermien so weit verringert werden kann, dass die Zeugungsfähigkeit eines derart behandelten Säugetiers zumindest vorübergehend vollständig terminiert werden kann. Bei diesem Verfahren ist insbesondere nachteilig, dass eine einmalige Anwendung nicht zu dem gewünschten Erfolg führt und daher mehrere Behandlungen notwendig sind. Darüber hinaus ist die Behandlungstemperatur mit 45°C so hoch, dass bei dem behandelten Säugetier mit hoher Wahrscheinlichkeit ein Schmerzreiz ausgelöst wird. Die hohe Behandlungstemperatur ist bei diesem Verfahren jedoch erforderlich, um ein ausreichendes Durchwärmen der Hoden, inklusive der die Spermien enthaltenden Nebenhoden, zu erreichen.

Beispiele für derartige Erwärmungsverfahren und Erwärmungsvorrichtungen zur Erwärmung der Nebenhoden sind in den Druckschriften US 5 063 939 A, EP 2 810 694 A1 und DE 20 2014 004 985 U1 beschrieben.

Aus der US 1 633 878 ist eine Vorrichtung zur Wärmbehandlung insbesondere der männlichen Geschlechtsorgane mit konkaven, drehbar gelagerten Greifplatten als Elektroden, die verstellbar an einer scharnierartigen Halterung angebracht sind, bekannt.

Um Gewebe lokal zu erwärmen, ist aus dem Bereich der Chirurgie beispielsweise die bipolare HF-Chirurgie bekannt. Bei diesem Verfahren wird über zwei Elektroden ein hochfrequenter Kontaktstrom in ein Gewebe eingeleitet. Die verwendeten Elektroden weisen dabei üblicherweise eine Größe von wenigen Millimetern auf. Dabei ist es das Ziel der HF-Chirurgie, das ausgewählte Gewebe so weit zu erhitzen, dass es koaguliert beziehungsweise dass durch ein Platzen der erhitzten Körperzellen ein Schnitt ausbildbar ist. Aufgrund des hohen lokal begrenzten Eintrags thermischer Energie in das zu behandelnde Gewebe sind die Verfahren der bipolaren HF-Chirurgie nicht geeignet, um empfindliche Organe, wie beispielsweise die Nebenhoden, lokal und um nur wenige Grad zu erwärmen.

Darüber hinaus sind aus dem Stand der Technik sogenannte Diathermie-Geräte bekannt. Diese Geräte weisen Antennen auf, mittels derer großflächig elektromagnetische Strahlung in organisches Gewebe eingebracht werden kann. Die dabei auftretende Erwärmung ist zwar gering genug, um eine Zerstörung von Gewebe zu verhindern, jedoch ist die Wirkung so großflächig, dass mittels derartiger Diathermie-Geräte das lokale Erwärmen der Nebenhoden ebenfalls nicht möglich ist.

Zusammenfassend kann festgestellt werden, dass es mit keinem der bekannten Verfahren und Vorrichtungen möglich ist, einen die Spermien enthaltenden Teil des Körpers eines Säugetiers zielgerichtet lokal zu erwärmen, ohne einen Schmerzreiz auszulösen und/oder Gewebe zu zerstören.

Als Aufgabe der vorliegenden Erfindung wird es daher angesehen, eine Vorrichtung zur Empfängnisverhütung zur Verfügung zu stellen, mittels derer ein lokal begrenzter, an der Spermienbildung beteiligter Gewebebereich eines Säugetiers nicht-invasiv erwärmbar ist.

Diese Aufgabe wird erfindungsgemäß dadurch gelöst, dass eine Kontrazeptionsvorrichtung zur Reduzierung einer Qualität von Spermien eines Säugetiers zur Verfügung gestellt wird, die eine Elektrodenanordnung mit zumindest einer ersten Elektrode und einer parallel zu der ersten Elektrode ausrichtbaren zweiten Elektrode, und einen HF-Generator zur Erzeugung einer hochfrequenten Wechselspannung aufweist, wobei die Elektroden derart mit dem HF-Generator in elektrische Wirkverbindung bringbar sind, dass die hochfrequente Wechselspannung zwischen der ersten Elektrode und der zweiten Elektroden anlegbar ist, wobei die Elektroden derart an eine Anatomie des Säugetiers angepasst und/ oder anpassbar sind, dass ein durch die HF-Spannung induzierter elektrischer Strom in zumindest einen Körperabschnitt des Säugetiers einleitbar ist, wobei in dem Körperabschnitt zumindest einer der Nebenhoden des Säugetiers zumindest abschnittsweise angeordnet ist und wobei durch den elektrischen Strom der Nebenhoden zumindest abschnittsweise erwärmbar ist, sodass die Qualität von in dem Nebenhoden enthaltenen Spermien derart einschränkbar ist, dass die Fertilität des Säugetiers vorübergehend eingeschränkt ist, und wobei die Kontrazeptionsvorrichtung zumindest eine Haltevorrichtung aufweist, wobei die Elektrodenanordnung derart an der Haltevorrichtung festlegbar ist, dass die erste Elektrode und die zweite Elektrode einander zugewandt und parallel zueinander ausgerichtet sind, wobei die Kontrazeptionsvorrichtung ein Abstandselement aufweist, wobei das Abstandselement dazu bestimmt ist, in einem Stützbereich an einem Oberflächenabschnitt des Körpers des Säugetiers anzuliegen, wobei das Abstandselement, die Elektrodenanordnung und die Haltevorrichtung derart miteinander in mechanischer Wirkverbindung sind, dass eine durch die Haltevorrichtung ausgeübte Haltekraft über das Abstandselement zumindest teilweise in dem Stützbereich in den Körper des Säugetiers einleitbar ist. Aufgrund des elektrischen Widerstands des zwischen den Elektroden angeordneten Gewebes wird die elektrische Energie des durch die HF-Spannung induzierten elektrischen Stroms zumindest teilweise in thermische Energie umgewandelt.

In einer vorteilhaften Ausführungsform der Erfindung sind die beiden Elektroden planare Elektroden. Geeignete planare Elektroden weisen typischerweise eine Stärke von mindestens 0,7 mmm, bevorzugt mindestens 1 mm auf.

Ohne sich auf eine Theorie einschränken zu wollen, wird davon ausgegangen, dass durch die in das Gewebe eingebrachte thermische Energie und einer daraus resultierenden Erhöhung einer Temperatur der Nebenhoden vor allem die Beweglichkeit der Spermien, insbesondere die Vorwärtsbeweglichkeit, so stark eingeschränkt wird, dass eine Befruchtung einer Eizelle nicht möglich ist. Besonders bevorzugt ist die erfindungsgemäße Kontrazeptionsvorrichtung derart ausgebildet, dass die Temperatur der Nebenhoden über eine normale Körpertemperatur hinaus erhöhbar ist. Besonders bevorzugt wird die Temperatur eines Teils des Nebenhodens, insbesondere der Nebenhodenschwanz (Cauda epidymidis) auf wenigstens 40 °C erwärmt. Dabei kann auch der Beginn des Samenleiters des Säugetiers mit erwärmt werden, wobei aber weitere Organe des Säugetiers, insbesondere der Hoden, von einer Erwärmung weitestgehend verschont bleiben. Die normale Körpertemperatur des Menschen liegt typischerweise zwischen 36,3°C und 37,4°C.

Es ist vorgesehen, dass mittels der erfindungsgemäßen Kontrazeptionsvorrichtung die Spermienqualität derart verringerbar ist, dass nach einer einmaligen Anwendung das behandelte Säugetier für mehrere Wochen nicht zeugungsfähig ist.

In Bezug auf die erfindungsgemäße Kontrazeptionsvorrichtung ist vorgesehen, dass bis zum tatsächlichen Erreichen der Zeugungsunfähigkeit stets auch ein Spülen des Reproduktionsapparates, insbesondere der Samenleiter, der Ductus Ejaculatroris und der Harnröhre erforderlich ist. Üblicherweise geschieht dies durch mehrmaliges Ejakulieren. Hierdurch werden die noch vorhandenen zeugungsfähigen Spermien aus dem Reproduktionsapparat ausgetragen.

Es ist vorteilhafterweise vorgesehen, dass die Elektroden eine Form aufweisen, die derart an eine Anatomie des zu behandelnden Säugetiers angepasst ist, dass gezielt Teile der Nebenhoden erwärmbar sind, wobei eine Erwärmung und damit eine eventuelle Beeinträchtigung anderer Organe weitestgehend verhindert ist. Besonders bevorzugt sind die Elektroden derart ausgestaltet, dass vorrangig ein distales Ende der Nebenhoden erwärmbar ist, wobei das distale Ende der Nebenhoden dasjenige Ende der Nebenhoden ist, an dem der Samenleiter beginnt.

Es ist darüber hinaus auch möglich und in weiterer Ausgestaltung der Erfindung vorgesehen, dass die Kontrazeptionsvorrichtung eine Elektrodenanordnung mit mehr als zwei Elektroden aufweist und wobei die mehr als zwei Elektroden gleichzeitig zur Behandlung jeweils eines Körperabschnitts einsetzbar sind. Durch eine derartige Ausgestaltung der Kontrazeptionsvorrichtung wird es ermöglicht, dass eine Energiemenge, die zur Erwärmung des Körperabschnitts in den Körperabschnitt eingebracht wird, besonders differenziert anpassbar ist. Um die Energiemenge besonders fein regeln zu können, ist es in weiterer vorteilhafter Ausgestaltung der Erfindung vorgesehen, dass die Elektrodenanordnung eine Vielzahl von Elektroden pro Hoden aufweisen kann.

Aufgrund der guten Regelbarkeit der Energiemenge, die durch die erfindungsgemäße Kontrazeptionsvorrichtung in den zu behandelnden Körperabschnitt eingetragen wird, wird durch eine Erwärmung des zu behandelnden Körperabschnitts bei dem Säugetier kein temperaturbasierter Schmerzreiz ausgelöst. Darüber hinaus ermöglicht der lokal begrenzte und zielgerichtete Eintrag der thermischen Energie, dass eine Anwendungsdauer der erfindungsgemäßen Kontrazeptionsvorrichtung, insbesondere im Vergleich zu den aus dem Stand der Technik bekannten Vorrichtungen, besonders kurz wählbar ist und trotzdem mit einer einmaligen Anwendung die Spermienqualität ausreichend reduzierbar ist, um eine vorübergehende Zeugungsunfähigkeit des behandelten Säugetiers zu erreichen.

Um eine elektrische Stimulation von in dem Körperabschnitt und/oder angrenzend an den Körperabschnitt angeordneten Nervenbahnen zu unterbinden, ist es erfindungsgemäß vorgesehen, dass Frequenzen der durch den HF-Generator erzeugten Wechselspannungen größer als 10 kHz sind. Dabei ist vorteilhafterweise vorgesehen, dass eine Einkopplung des hochfrequenten Stroms bei der erfindungsgemäßen Kontrazeptionsvorrichtung transkutan erfolgt, insbesondere durch eine Hodensackhaut des Hodensacks des Säugetiers.

Durch die bevorzugt vorgesehene Frequenz der Wechselspannung von über 10 kHz wird auch erreicht, dass eine direkte Stimulation von Schmerzrezeptoren verhindert ist. Damit ist bei der erfindungsgemäßen Kontrazeptionsvorrichtung auch verhindert, dass ein direkt durch die Wechselspannung induzierter Schmerzreiz ausgelöst wird.

Die Elektroden der erfindungsgemäßen Kontrazeptionsvorrichtung sind bevorzugt deutlich größer als die, die beispielsweise bei der HF-Chirurgie verwendet werden. Sie sind jedoch kleiner als die Antennen, die bei den aus dem Stand der Technik bekannten Diathermie-Geräten zur Anwendung kommen.

Erfindungsgegemäß ist vorgesehen, dass die Kontrazeptionsvorrichtung zumindest eine Haltevorrichtung aufweist, wobei die Elektrodenanordnung derart an der Haltevorrichtung festlegbar sind, dass die erste Elektrode und die zweite Elektrode einander zugewandt und parallel zueinander ausgerichtet sind. Die parallele Ausrichtung der jeweils zueinander gehörigen Elektroden ist insbesondere wichtig, um eine gleichmäßige Erwärmung des zwischen den Elektroden befindlichen Körperabschnitts zu ermöglichen und eine ungewollte, zu hohe oder zu geringe Erwärmung von einzelnen Gewebebereichen zu minimieren.

Vorteilhafterweise kann es darüber hinaus vorgesehen sein, dass die Haltevorrichtung derart an die Anatomie des Säugetiers angepasst oder anpassbar ist, dass ein Verrutschen der Elektroden während eines Betriebs der erfindungsgemäßen Kontrazeptionsvorrichtung verhindert ist. Derartige Vorrichtungen sind aus anderen Bereichen der Medizin, insbesondere aus der Chirurgie hinlänglich bekannt, beispielsweise aus dem Bereich der Hirnchirurgie.

Erfindungsgemäß ist vorgesehen, dass die Haltevorrichtung zumindest ein Abstandselement aufweist, wobei das Abstandselement bei bestimmungsgemäßer Verwendung der Kontrazeptionsvorrichtung in einem Stützbereich an einem Oberflächenabschnitt des Körpers des Säugetiers anliegt. Dabei ist vorgesehen, dass das Abstandselement, die Elektrodenanordnung und die Haltevorrichtung derart miteinander in mechanische Wirkverbindung gebracht sind, dass eine durch die Haltevorrichtung ausgeübte Haltekraft über das Abstandselement zumindest teilweise in dem Stützbereich in den Körper des Säugetiers einleitbar ist.

Besonders bevorzugt ist die Haltekraft vollständig über das Abstandselement in dem Stützbereich in den Körper des Säugetiers einleitbar. Vorteilhafterweise ist vorgesehen, dass der Stützbereich nicht dem Körperabschnitt zugeordnet ist, in dem Hoden oder der Nebenhoden des Säugetiers angeordnet sind. Hierdurch wird erreicht, dass ein eventuell durch die Haltekraft der Haltevorrichtung ausgelöster Schmerzreiz minimierbar ist.

Es ist auch möglich und erfindungsgemäß vorgesehen, dass die Kontrazeptionsvorrichtung derart an eine Anatomie des Säugetiers angepasst sein kann, dass der Stützbereich bei bestimmungsgemäßer Verwendung der Kontrazeptionsvorrichtung in einem der Verwachsungslinie an der Unterseite des Hodensacks (Raphe scroti) zuordenbaren Abschnitt der Hodensackhaut angeordnet ist.

Es ist besonders bevorzugt vorgesehen, dass die Haltevorrichtung derart ausgebildet ist, dass mit ihr ein Hoden und der dem Hoden zugeordnete Nebenhoden von derjenigen Körperseite her umgreifbar ist, der der Hoden zugeordnet ist, also ein auf der linken Körperseite angeordneter Hoden von links und ein auf der rechten Körperseite angeordneter Hoden von rechts.

Bevorzugt weist die Haltevorrichtung zumindest ein erstes und ein zweites Abstandselement auf, wobei bei bestimmungsgemäßer Verwendung der Kontrazeptionsvorrichtung die Raphe scroti, beziehungsweise ein unmittelbar an die Raphe scroti angrenzender Abschnitt des Hodensacks zwischen den Abstandselementen angeordnet ist und den Stützbereich bildet.

In weiterer vorteilhafter Ausgestaltung der Erfindung weist die Haltevorrichtung ein Gelenk auf, welches einen Federmechanismus aufweist, durch den zwischen den Abstandselementen eine Haltekraft zwischen 1,5 N und 4 N erzeugbar ist. Die Haltekraft kann beispielsweise durch eine Schenkelfeder ausgeübt werden, die ein Drehmoment zwischen 0,06 Nm und 0,16 Nm erzeugt.

Eine weitere vorteilhafte Ausgestaltung der Erfindung sieht vor, dass die Kontrazeptionsvorrichtung zumindest einen Temperatursensor aufweist, wobei mittels des Temperatursensors eine Oberflächentemperatur eines dem Körperabschnitt zuordenbaren Körperoberflächenbereichs bestimmbar ist. Eine Bestimmung der Oberflächentemperatur ist so auf einfache Art und Weise möglich. Der Temperatursensor kann beispielsweise nach dem Prinzip eines Berührungsthermometers arbeiten und einen Heißleiter, einen Kaltleiter, einen Festkörperschaltkreis, eine Diode, einen Schwingquarz oder ein ähnliches Bauelement zur Bestimmung der Oberflächentemperatur aufweisen.

Alternativ kann es vorgesehen sein, dass der Temperatursensor nach dem Prinzip eines berührungslos messenden Thermometers ausgebildet ist. Hierzu kann er beispielsweise ein Pyrometer umfassen, mittels dessen eine Infrarotstrahlung messbar ist.

In noch weiterer vorteilhafter Ausgestaltung der Erfindung ist der Temperatursensor mit einer Anzeigevorrichtung gekoppelt oder koppelbar, wobei die Anzeigevorrichtung derart ausgebildet ist, dass Sensordaten auswertbar und anzeigbar sind. Die Anzeigevorrichtung kann dazu sowohl analoge Anzeigemittel, beispielsweise Zeiger, als auch digitale Anzeigemittel, beispielsweise ein LCD-Display, umfassen. Durch eine derartige Ausgestaltung der erfindungsgemäßen Kontrazeptionsvorrichtung wird es ermöglicht, dass ein Benutzer der Kontrazeptionsvorrichtung den Verlauf der Behandlung auf der Grundlage der durch den Temperatursensor ermittelten Temperatur verfolgen kann.

Bei einer vorteilhaften Umsetzung des Erfindungsgedankens ist es vorgesehen, dass die Kontrazeptionsvorrichtung signalleitend mit einer externen Datenverarbeitungseinrichtung in Wirkverbindung gebracht und/ oder bringbar sein kann. Eine signalleitende Verbindung kann dabei sowohl mittels eines Kabels, als auch mittels einer Funkverbindung hergestellt und/ oder herstellbar sein. Besonders bevorzugt ist die externe Datenverarbeitungseinrichtung ein Mobiltelefon, ein Tabletcomputer, ein Laptop, ein Arbeitsplatzcomputer oder ähnliches.

Bei einer besonders vorteilhaften Umsetzung des Erfindungsgedankens ist es vorgesehen, dass der Temperatursensor in zumindest eine der Elektroden integriert ist und dass mittels des Temperatursensors eine Elektrodentemperatur erfassbar ist.

Bei einer besonders vorteilhaften Ausgestaltung der erfindungsgemäßen Kontrazeptionsvorrichtung ist vorgesehen, dass die Kontrazeptionsvorrichtung ein Temperierungselement aufweist, wobei das Temperierungselement bei bestimmungsgemäßer Verwendung der Kontrazeptionsvorrichtung mit dem Körperabschnitt in thermische Wirkverbindung bringbar ist und wobei mittels des Temperierungselements thermische Energie in den Körperabschnitt einleitbar und/oder aus dem Körperabschnitt abführbar ist. Vorteilhafterweise ist es vorgesehen, dass die Elektroden derart ausgestaltet sind, dass sie thermische Energie von der Körperoberfläche, mit der sie in Kontakt stehen, abführen und insbesondere eine gleichmäßige Temperaturverteilung in einem Kontaktbereich begünstigen.

Um eine derartige Wärmeverteilung zu erreichen, kann es vorteilhafterweise vorgesehenen sein, dass die Elektroden aus einem Material gefertigt sind, das eine hohe Wärmeleitfähigkeit aufweist. Besonders geeignete Materialien zur Fertigung der Elektroden sind insbesondere Silber, Kupfer, Aluminium sowie Legierungen mit diesen Metallen.

Darüber hinaus ist es auch möglich und in Ausgestaltung der der Erfindung von Vorteil, dass die Elektroden aus einem nichtmetallischen Werkstoff, beispielsweise einer Spezialkeramik, einem elektrisch leitfähigen Polymer oder Ähnlichem gefertigt sein können.

Besonders bevorzugt sind Elektrodenmaterialien mit einer Wärmeleitfähigkeit von mindestens 200 W·m⁻¹·K⁻¹, ganz besonders bevorzugt mehr als 300 W ·m⁻¹·K⁻¹. Durch eine hohe Wärmeleitfähigkeit wird in den Elektroden vorteilhafterweise auch eine besonders gleichmäßige Wärmeverteilung erreicht.

Damit die Elektroden der erfindungsgemäßen Kontrazeptionsvorrichtung die thermische Energie von der Körperoberfläche, mit der sie in Kontakt stehen, abführen und insbesondere eine gleichmäßige Temperaturverteilung in einem Kontaktbereich begünstigen, ist es vorgesehen, dass die Elektroden ein Material aufweisen, das eine hohe Wärmeleitfähigkeit aufweist und gleichzeitig eine Mindeststärke besitzt. In einer besonders vorteilhaften Ausführungsform der Erfindung sind die Elektroden aus einem Material gefertigt, das ein Produkt aus Wärmeleitfähigkeit und Dicke von mindestens 3,8 W·K⁻¹ aufweist.

Bei einer besonders vorteilhaften Ausgestaltung der erfindungsgemäßen Kontrazeptionsvorrichtung ist vorgesehen, dass eine Kühlung des Körperabschnitts unter Ausnutzung einer Wärmespeicherkapazität der Elektroden erfolgt. Hierzu ist es beispielsweise möglich, dass die Elektroden einen Wärmespeicherabschnitt aufweisen, der beispielsweise als ein verdickter Abschnitt des Elektrodenmaterials ausgebildet sein kann. Es ist auch möglich, dass in dem Wärmespeicherabschnitt ein Material angeordnet sein kann, dass eine besonders hohe Wärmespeicherkapazität aufweist, beispielsweise Wasser. Darüber hinaus ist es auch möglich und erfindungsgemäß vorgesehen, dass die Elektroden passive Kühlelemente, beispielsweise Kühlrippen, aufweisen können, mittels derer in die Elektroden eingeleitete thermische Energie an eine Umgebung abgebbar ist.

Eine vorteilhafte Umsetzung des Erfindungsgedankens sieht vor, dass das Temperierungselement zumindest abschnittsweise als ein elektrisches Heizelement ausgebildet ist. Es ist vorgesehen, dass das elektrische Heizelement beispielsweise eine Spule, ein keramisches Heizelement, einen elektrischen Widerstand, bipolare Transistoren, Feldeffekttransistoren oder Ähnliches aufweisen kann. Eine derartige Ausgestaltung ist insbesondere vorteilhaft, wenn ein Vorwärmen des zu behandelnden Körperabschnitts und/oder der Elektroden erforderlich ist. Hierdurch ist insbesondere die Behandlungsdauer mit der erfindungsgemäßen Kontrazeptionsvorrichtung verkürzbar.

Es ist auch möglich und erfindungsgemäß vorgesehen, dass das Temperierungselement einen elektrothermischen Wandler, insbesondere ein Peltierelement, aufweisen kann.

Eine vorteilhafte Ausgestaltung der Erfindung sieht vor, dass das Temperierungselement Fluidleitungen aufweist, durch die ein Fluid leitbar ist, wobei das Temperierungselement derart an den Körperabschnitt angepasst ist, dass bei bestimmungsgemäßer Verwendung der Kontrazeptionsvorrichtung ein Wärmetransport zwischen dem Körperabschnitt und dem Fluid ermöglicht ist. Es ist vorteilhafterweise vorgesehen, dass die Fluidleitungen mit weiteren Elementen zur Ausbildung eines Temperierungskreislaufs verbunden sein können, insbesondere mit Pumpen, Radiatoren, Filtern, Heizelementen, Kühlelementen oder Ähnlichem. Es ist darüber hinaus vorgesehen, dass mittels des Fluids sowohl Wärme in den Körperabschnitt eintragbar, als auch aus dem Körperabschnitt abführbar sein kann.

Bei einer vorteilhaften Ausgestaltung der erfindungsgemäßen Kontrazeptionsvorrichtung ist vorgesehen, dass die Kontrazeptionsvorrichtung eine Regelungseinrichtung aufweist, wobei die Regelungseinrichtung derart mit dem HF-Generator in Wirkverbindung gebracht/bringbar ist, dass mittels der Regelungseinrichtung zumindest die durch den HF-Generator erzeugte Wechselspannung regelbar ist.

Bei einer vorteilhaften Ausgestaltung der erfindungsgemäßen Kontrazeptionsvorrichtung ist vorgesehen, dass auf der Regelungseinrichtung eine Regel hinterlegt ist, anhand derer eine zeitlich definierte Regelung der HF-Spannung erfolgt. So ist es beispielsweise möglich, dass an verschiedene Säugetiere angepasste Profile vorgesehen sein können, anhand derer die HF-Spannung durch die Regelungseinrichtung geregelt wird.

Darüber hinaus ist es auch möglich, dass die auf der Regelungseinrichtung hinterlegte Regel mittels externer Daten an ein spezifisches Säugetier anpassbar sein kann, beispielsweise indem Daten des Säugetiers, etwa Alter, Größe, Gewicht oder Ähnliches, über eine Schnittstelle, beispielsweise über ein Eingabegerät, in die Regelungseinrichtung eingegeben werden. Die Schnittstelle kann auch eine Verbindung zu einem Datennetzwerk, zu Datenspeichern oder ähnlichem aufweisen.

Bei einer besonders vorteilhaften Ausgestaltung der Erfindung ist es vorgesehen, dass die Regelungseinrichtung mittels der externen Datenverarbeitungseinrichtung steuerbar ist. Die externe Datenverarbeitungseinrichtung kann dazu signalleitend mit der Regelungseinrichtung in Wirkverbindung gebracht und/ oder bringbar sein kann, wobei eine derartige Verbindung sowohl mittels eines Kabels als auch mittels einer Funkverbindung hergestellt und/ oder herstellbar sein kann.

Besonders bevorzugt ist die externe Datenverarbeitungseinrichtung ein Mobiltelefon, ein Tabletcomputer, ein Laptop, ein Arbeitsplatzcomputer oder ähnliches. Es ist dabei auch möglich und erfindungsgemäß vorgesehen, dass die Regel auf der externen Datenverarbeitungseinrichtung hinterlegt und/ oder hinterlegbar sein kann, beispielsweise als Bestandteil eines Softwaremoduls.

Eine vorteilhafte Umsetzung des Erfindungsgedankens sieht vor, dass die Regelungseinrichtung eine Datenverarbeitungseinrichtung aufweist, wobei die Datenverarbeitungseinrichtung signal- und/ oder datenleitend mit dem Temperatursensor verbunden/ verbindbar ist und wobei mittels der Datenverarbeitungseinrichtung aufgrund von Temperatursensordaten und anhand einer in der Datenverarbeitungseinrichtung hinterlegten Regel eine Nebenhodentemperatur bestimmbar ist. Der Temperatursensor der erfindungsgemäßen Kontrazeptionsvorrichtung ist derart ausgebildet, dass er die Oberflächentemperatur eines dem Körperabschnitt zuordenbaren Körperoberflächenbereichs erfasst. Um die Nebenhodentemperatur zu bestimmen, ist es deshalb vorteilhafterweise vorgesehen, dass mittels der Regel aus der Oberflächentemperatur die Nebenhodentemperatur extrapolierbar ist.

Eine vorteilhafte Ausgestaltung der Erfindung sieht vor, dass eine Regelung der Wechselspannung durch die Regelungseinrichtung zumindest teilweise auf der Grundlage von Temperatursensordaten des Temperatursensors erfolgt. Durch eine derartige Ausgestaltung der erfindungsgemäßen Kontrazeptionsvorrichtung wird es ermöglicht, dass eine Behandlung entsprechend anatomischer Besonderheiten verschiedener Säugetiere durchführbar ist. Insbesondere ist vorteilhafterweise feststellbar, ob eine ausreichende Erwärmung der Spermien erfolgt ist.

Darüber hinaus ist bei dieser Ausführungsform der Kontrazeptionsvorrichtung vorteilhafterweise das Risiko dafür minimiert, dass durch eine zu starke Erwärmung des Körperabschnitts eine Schädigung, insbesondere auch eine dauerhafte Schädigung, des Körperabschnitts auftritt.

Bei einer besonders vorteilhaften Ausgestaltung der erfindungsgemäßen Kontrazeptionsvorrichtung ist vorgesehen, dass durch die Regelungseinrichtung auch das Temperierungselement regelbar ist.

Es ist auch möglich und erfindungsgemäß vorgesehen, dass die erfindungsgemäße Kontrazeptionsvorrichtung derart ausgestaltet sein kann, dass eine gleichzeitige Behandlung beider Nebenhoden des Säugetiers durchführbar ist. Hierzu ist es insbesondere vorgesehen, dass die Kontrazeptionsvorrichtung mehrere HF-Generatoren, Haltevorrichtungen, Elektrodenanordnungen, Temperatursensoren usw. aufweisen kann.

Darüber hinaus ist es erfindungsgemäß ebenfalls vorgesehen, dass eine erweiterte Kontrazeptionsvorrichtung, insbesondere mehrere HF-Generatoren, Haltevorrichtungen, Elektrodenanordnungen und Temperatursensoren umfassen kann, wobei mittels der erweiterten Kontrazeptionsvorrichtung der gesamte Hoden des Säugetiers, bevorzugt einschließlich des Nebenhodens, erwärmt werden kann.

Nachfolgend ist ein Kontrazeptionsverfahren zur Reduzierung der Qualität von Spermien eines Säugetiers mit Hodenabstieg durch ein lokales Erwärmen zumindest eines Nebenhodens des Säugetiers mittels einer der zuvor beschriebenen Kontrazeptionsvorrichtungen dargestellt. Das Verfahren ist auch zur Anwendung beim Menschen geeignet.

Bei dem Verfahren ist vorgesehen, dass das Erwärmen der Nebenhoden des Säugetiers mittels einer zuvor beschriebenen Kontrazeptionsvorrichtung erfolgt, wobei zunächst in einem Positionierungsschritt die Kontrazeptionsvorrichtung an zumindest einem Körperabschnitt des Säugetiers positioniert wird, wobei in dem Körperabschnitt zumindest einer der Nebenhoden des Säugetiers zumindest abschnittsweise angeordnet ist, wobei in einem auf den Positionierungsschritt folgenden Erwärmungsschritt die HF-Wechselspannung erzeugt und ein durch die HF-Wechselspannung induzierte elektrischer Strom in den Körperabschnitt eingeleitet wird, wodurch die Nebenhoden zumindest abschnittsweise erwärmt werden, sodass die Qualität von in dem Nebenhoden enthaltenen Spermien derart einschränkt wird, dass die Fertilität des Säugetiers vorübergehend eingeschränkt wird. Besonders bevorzugt wird die Temperatur der Nebenhoden in dem Erwärmungsschritt über die normale Körpertemperatur des Säugetiers hinaus erhöht.

Bei einer besonders vorteilhaften Umsetzung des Verfahrens ist es vorgesehen, dass in dem Positionierungsschritt der Hoden mitsamt dem zu behandelnden Nebenhoden derart gedreht wird, dass der zu behandelnde Nebenhoden in Richtung der Raphe scroti verlagert ist. Durch eine derartige Ausgestaltung des Verfahrens wird insbesondere ein Umgreifen des Hodens und des Nebenhodens mit der Kontrazeptionsvorrichtung erleichtert. Bevorzugt beträgt ein Verdrehwinkel des Hodens zwischen 70° und 110°, besonders bevorzugt 90°.

Um eine ausreichende Erwärmung der Nebenhoden zu gewährleisten und deren Überhitzung auszuschließen, ist es bei einer vorteilhaften Ausgestaltung des Verfahrens vorgesehen, dass das Verfahren mit einer der zuvor beschriebenen Kontrazeptionsvorrichtung erfolgt, die einen Temperatursensor aufweist, mittels dessen die Oberflächentemperatur des dem Körperabschnitt zuordenbaren Körperoberflächenbereichs bestimmbar ist.

Besonders bevorzugt wird das Verfahren mittels einer Kontrazeptionsvorrichtung durchgeführt, die eine Regelungseinrichtung mit einer Datenverarbeitungseinrichtung aufweist, wobei die Datenverarbeitungseinrichtung signal-und/oder datenleitend mit dem Temperatursensor verbunden/ verbindbar ist und wobei mittels der Datenverarbeitungseinrichtung aufgrund von Temperatursensordaten und anhand einer in der Datenverarbeitungseinrichtung hinterlegten Regel die Nebenhodentemperatur bestimmbar ist. Dies ist insbesondere vorteilhaft, da die Oberflächentemperatur bei bestimmungsgemäßer Anwendung des Verfahrens prinzipbedingt geringer ist als die Nebenhodentemperatur.

Bei einer weiteren vorteilhaften Ausgestaltung des Kontrazeptionsverfahrens erfolgt ein permanenter Abgleich der Oberflächentemperatur und/ oder der Nebenhodentemperatur mit zuvor festgelegten Maximal- und/ oder Zielwerten, wobei sowohl bei Erreichen eines Maximal- als auch eines Zielwerts eine Beendigung der Anwendung vorgesehen sein kann. Es ist dabei auch möglich, dass bei dem Erreichen des Zielwerts die Anwendung über eine Behandlungsdauer hinweg fortgesetzt und nach Ablauf der Behandlungsdauer beendet wird.

Bei einer vorteilhafte Ausgestaltung des Kontrazeptionsverfahrens ist es vorgesehen, dass in einem vor dem Positionierungsschritt erfolgenden Vorbereitungsschritt ein Elektrodengel auf eine dem Körperabschnitt zuordenbare Körperoberfläche und/ oder zumindest eine der Elektroden aufgetragen wird, wobei durch das Elektrodengel ein elektrischer und/ oder thermischer Widerstand zwischen den Elektroden und dem Körperabschnitt verringert ist.

Um den Kontakt zwischen den Elektroden und der Hodensackhaut zu verbessern, ist es vorgesehen, dass ein Elektrodengel verwendet wird, das eine ausreichende Viskosität besitzt, um zwischen Elektrode und Hodensackhaut zu haften. Die elektrischen Eigenschaften des Elektrodengels sind dabei vorteilhafterweise an die elektrischen Eigenschaften der Hodensackhaut und der Nebenhoden angepasst, um ein Risiko von partiellen Überhitzungen zu minimieren. Dabei ist es vorteilhafterweise auch vorgesehen, dass das Elektrodengel eine thermische Leitfähigkeit aufweisen kann, die einen Wärmetransport zwischen der Hodensackhaut und den Elektroden begünstigt.

Es ist darüber hinaus vorgesehen, dass mittels des Verfahrens sowohl eine simultane als auch eine serielle Behandlung der Nebenhoden des Säugetiers erfolgen kann.

Nachfolgend werden einige Ausführungsbeispiele der erfindungsgemäßen Kontrazeptionsvorrichtung sowie ihrer möglichen Anwendung näher erläutert, die in der Zeichnung dargestellt sind. Es zeigen:
Figuren 1, 2, 5 und 6 schematische Darstellungen von Ausführungsformen der erfindungsgemäßen Kontrazeptionsvorrichtung,
Figur 3 eine schematische Darstellung einer Elektrode aus Fig. 1,
Figur 4 ein schematisch dargestelltes Blockschaltbild einer Ausführungsform der erfindungsgemäßen Kontrazeptionsvorrichtung,
Figur 7 eine schematische Darstellung des zwischen den Elektroden einer erfindungsgemäßen Kontrazeptionsvorrichtung wirkenden elektrischen Feldes,
Figur 8 eine schematische Darstellung einer Elektrode aus Fig. 2 und
Figur 9 eine schematische Darstellung eines Kontrazeptionsverfahrens, das mittels der Kontrazeptionsvorrichtung durchführbar ist.

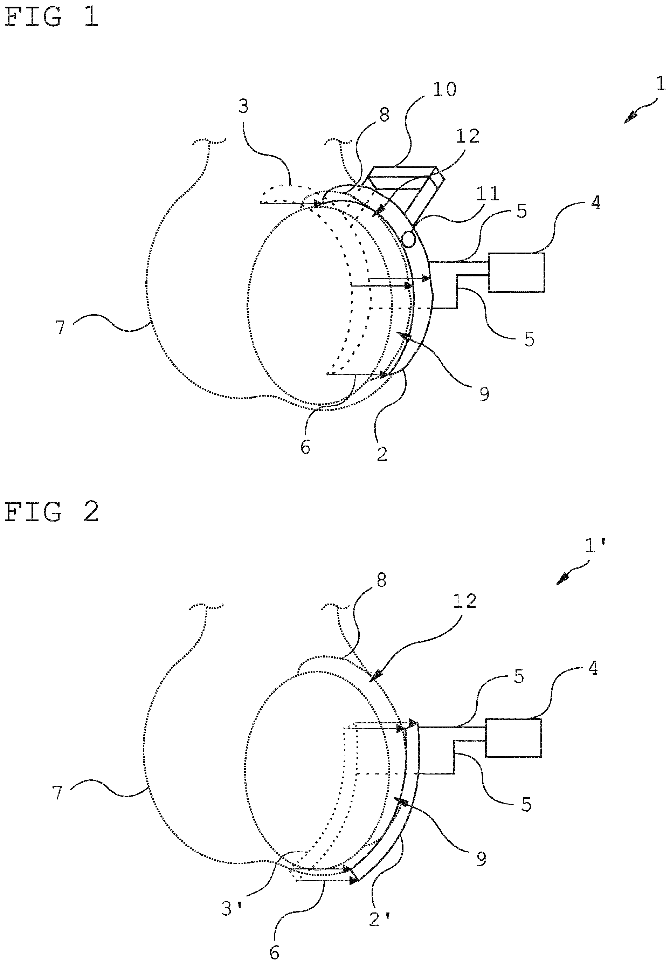

In Figur 1 ist eine schematische Darstellung einer erfindungsgemäßen Kontrazeptionsvorrichtung 1 gezeigt. Die Kontrazeptionsvorrichtung 1 weist eine Elektrodenanordnung mit einer ersten Elektrode 2 und einer zweiten Elektrode 3, sowie einen HF-Generator 4 zur Erzeugung einer hochfrequenten Wechselspannung auf. Die erste Elektrode 2 und die zweite Elektrode 3 sind mittels jeweils eines Elektrodenkabels 5 an den HF-Generator 4 angeschlossen. Der HF-Generator 4 bildet auf den Elektroden 2, 3 jeweils alternierende Pole aus, wobei eine Polung der ersten Elektrode 2 stets invers zu einer Polung der zweiten Elektrode 3 ist. Zwischen den beiden Elektroden 2, 3 ist so die durch den HF-Generator 4 erzeugte Wechselspannung angelegt. Die Wechselspannung ist in Figur 1 durch exemplarisch gezeigt Feldlinien 6 dargestellt, wobei die gezeigten Feldlinien 6 schematisch den Bereich darstellen, in dem zwischen den beiden Elektroden 2, 3 ein im Wesentlichen homogenes elektrisches Feld ausgebildet ist.

Die Elektroden 2, 3 der dargestellten Kontrazeptionsvorrichtung 1 sind derart an einen Hodensack 7 eines Säugetiers (nicht dargestellt und bezeichnet) angepasst, dass in dem Hodensack 7 angeordnete Nebenhoden 8 des Säugetiers überwiegend in dem homogenen Teil des zwischen den Elektroden 2, 3 ausgebildeten elektrischen Feldes angeordnet sind. Entlang der Feldlinien 6 wird in den Nebenhoden 8 ein elektrischer Strom induziert. Aufgrund des elektrischen Widerstands des von dem elektrischen Strom durchflossenen Gewebes 9 wird das Gewebe 9 erwärmt. In dem Nebenhoden 8 befinden sich Spermien, die zusammen mit dem Nebenhoden erwärmt werden. Durch das Erwärmen wird die Qualität der in dem Nebenhoden 8 enthaltenen Spermien derart verringert, dass die Fertilität des Säugetiers vorübergehend eingeschränkt ist.

Die dargestellte Ausführungsform der Kontrazeptionsvorrichtung 1 weist eine Haltevorrichtung 10 auf, an der die Elektroden 2, 3 festgelegt sind. Durch die Haltevorrichtung 10 sind die Elektroden 2, 3 derart gehalten, dass sie einander zugewandt und parallel zueinander ausgerichtet sind. Die parallele Ausrichtung der Elektroden 2, 3 zueinander führt dazu, dass das zwischen den Elektroden 2, 3 ausgebildete elektrische Feld abschnittsweise homogen ist, wie in Figur 1 durch die Feldlinien 6 dargestellt ist.

Die gezeigte Kontrazeptionsvorrichtung 1 weist auch einen Temperatursensor 11 auf. Der Temperatursensor 11 ist im Bereich der ersten Elektrode 2 an einem Abschnitt der Hodensackhaut 12 des Hodensacks 7 angeordnet. Mittels des Temperatursensors 11 ist die aktuelle Oberflächentemperatur der Hodensackhaut 12 bestimmbar. Aufgrund der räumlichen Nähe zwischen dem Temperatursensor 11 und den Nebenhoden 8 ist auf der Grundlage der durch den Temperatursensor 11 bestimmten Oberflächentemperatur eine Nebenhodentemperatur bestimmbar.

Figur 2 zeigt eine schematische Darstellung einer alternativen Ausführungsform einer erfindungsgemäßen Kontrazeptionsvorrichtung 1`. Die Kontrazeptionsvorrichtung 1' weist eine Elektrodenanordnung mit einer ersten Elektrode 2' und einer zweiten Elektrode 3', sowie einen HF-Generator 4 zur Erzeugung einer hochfrequenten Wechselspannung auf. Die Elektroden 2', 3' sind parallel zueinander derart an dem Nebenhoden 8 festgelegt, dass nur ein distales Ende des Nebenhodens 8 zwischen den Elektroden 2' und 3' angeordnet ist. Bei angelegter Wechselspannung zwischen den Elektroden 2', 3' wird somit gezielt das distale Ende des Nebenhodens 8 erwärmt. Insbesondere wird die Cauda epididymidis erwärmt, in der reife Spermien enthalten sind. Andere Organe und insbesondere der benachbarte Hoden werden nicht oder nur sehr geringfügig erwärmt. Dadurch werden mögliche unerwünschte Auswirkungen auf die im Hoden stattfindende Spermienproduktion und auf den Hormonhaushalt bei dem behandelten Säugetier vermieden. Es hat sich überraschend gezeigt, dass eine Erwärmung der reifen Spermien in der Cauda epididymidis genügt, um die Motilität der Spermien derart zu reduzieren, dass eine vorübergehende Zeugungsunfähigkeit des behandelten Säugetiers erreicht wird.

Die Elektroden 2' und 3' der in Fig. 2 dargestellten Kontrazeptionsvorrichtung 1' sind beim Betrieb der Kontrazeptionsvorrichtung entlang ihrer Längsseiten einander zugewandt und parallel zueinander ausgerichtet. Die Elektroden 2', 3' weisen eine gleichmäßige Breite und Stärke auf. Dadurch entsteht im Betrieb der erfindungsgemäßen Kontrazeptionsvorrichtung zwischen den Elektroden 2' und 3' ein besonders homogenes elektrisches Feld. In Längsrichtung weisen die Elektroden 2', 3' eine gebogene Form auf, die an den gebogenen Verlauf des Nebenhodens entlang des Hodens angepasst ist. Der in dem Bereich zwischen den Elektroden 2' und 3' angeordnete Abschnitt des Nebenhodens 8 wird in dieser Anordnung besonders gleichmäßig erwärmt und die Bildung von Hot Spots wird vermieden. Die Kanten der Elektroden können abgerundet sein, um bei der Anwendung ein Einschneiden in das angrenzende Körpergewebe zu vermeiden.

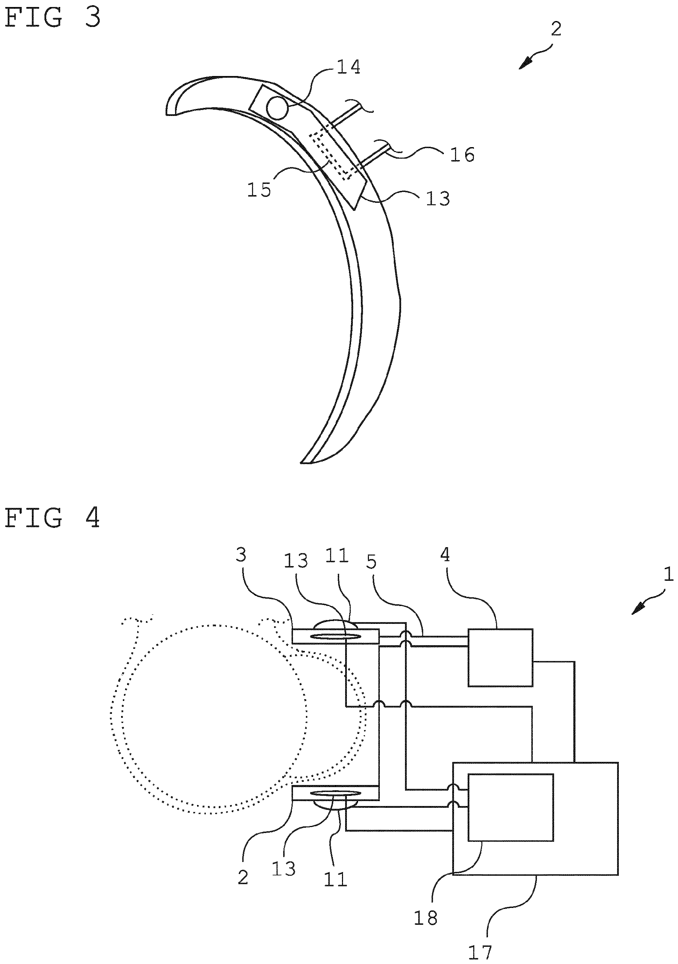

In Figur 3 ist eine schematische Darstellung der ersten Elektrode 2 der in Figur 1 gezeigten Kontrazeptionsvorrichtung 1 gezeigt. Die erste Elektrode 2 weist ein Temperierungselement 13 auf. Das Temperierungselement 13 ist mit der Hodensackhaut 12 in thermische Wirkverbindung bringbar, sodass mittels des Temperierungselements 13 über die Hodensackhaut 12 thermische Energie in den Hodensack 7 einleitbar und/ oder aus dem Hodensack 7 abführbar ist.

Das Temperierungselement 13 umfasst ein als Heizspule ausgebildetes elektrisches Heizelement 14. Das Temperierungselement 13 weist auch eine Fluidleitung 15 auf, durch die ein Fluid leitbar ist, mittels dessen thermische Energie zuführbar oder ableitbar ist. Die Fluidleitung 15 ist über ein abschnittsweise dargestelltes Schlauchsystem 16 mit einer nicht dargestellten Umwälz- und Temperierungseinrichtung verbunden, mittels der das Fluid förderbar ist und eine Fluidtemperatur einstellbar ist.

In Figur 4 ist ein schematisch dargestelltes Blockschaltbild einer weiteren Ausführungsform der erfindungsgemäßen Kontrazeptionsvorrichtung 1 gezeigt. Die Kontrazeptionsvorrichtung 1 weist eine Regelungseinrichtung 17, einen HF-Generator 4, eine erste Elektrode 2, eine zweite Elektrode 3, zwei Temperatursensoren 11, die jeweils wärmeleitend mit einer der Elektroden 2, 3 verbunden sind, und zwei Temperierungselemente 13 auf, die jeweils in einer der Elektroden 2, 3 ausgebildet sind. Die Regelungseinrichtung 17 weist eine Datenverarbeitungseinrichtung 18 auf, die datenleitend mit dem Temperatursensor 11 verbunden ist. Die Elektroden 2, 3, der Temperatursensor 11 und das Temperierungselement sind an einem schematisch dargestellten Hodensack 7 angeordnet.

Die Regelungseinrichtung 17 ist mit dem HF-Generator 4 in Wirkverbindung gebracht, sodass mittels der Regelungseinrichtung 17 die durch den HF-Generator 4 erzeugte Wechselspannung regelbar ist. In der Datenverarbeitungseinrichtung 18 ist eine Regel hinterlegt, anhand der auf der Grundlage von Temperatursensordaten des Temperatursensors 11 die Nebenhodentemperatur bestimmbar ist. Die Regelung der durch den HF-Generator 4 erzeugten Wechselspannung ist bei der dargestellten Kontrazeptionsvorrichtung 1 auf der Grundlage der Temperatursensordaten des Temperatursensors ermöglicht.

Die Regelungseinrichtung 17 ist auch mit den Temperierungselementen 13 verbunden, sodass die Nebenhodentemperatur mittels der Temperierungselemente 13 durch die Regelungseinrichtung 17 der Kontrazeptionsvorrichtung 1 beeinflussbar ist. Vorzugsweise erfolgt durch die Regelungseinrichtung 17 ein Abgleich der Temperatursensordaten, welche die Temperatursensoren 11 an den Elektroden 2 und 3 messen. Bei Abweichungen zwischen den jeweiligen Messwerten kann ein Temperaturausgleich durch Kühlung oder Erwärmung einer der beiden Elektroden 2, 3 durch Aktivierung des jeweiligen Temperierungselements 13 erfolgen. Der Abgleich der Temperatursensordaten kann auch zur Überprüfung der Funktionsfähigkeit der Temperatursensoren 11 genutzt werden.

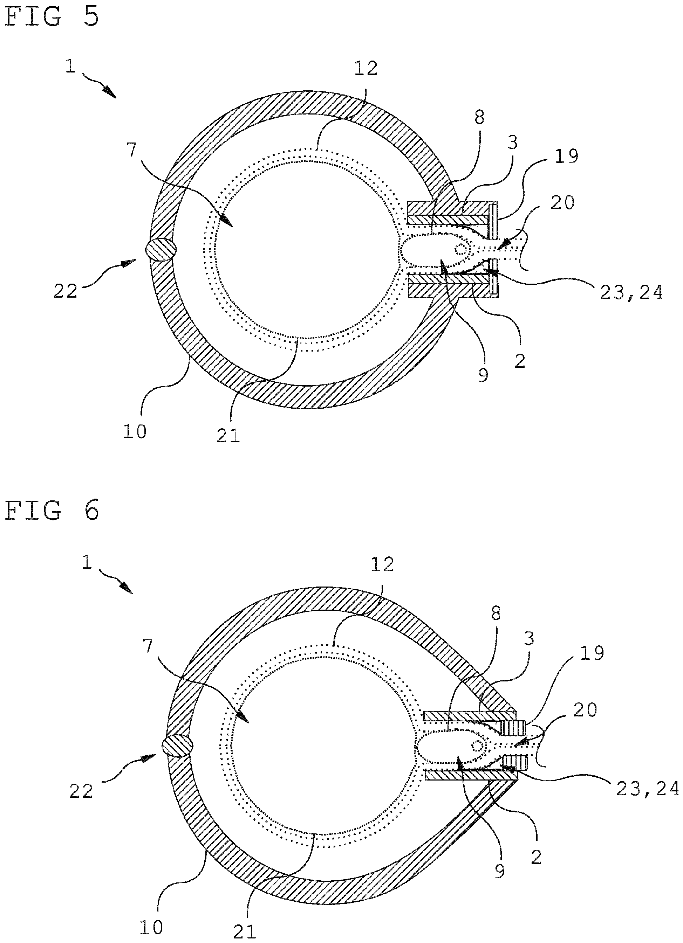

In Figur 5 ist eine schematisch dargestellte Schnittansicht einer Ausführungsform der erfindungsgemäßen Kontrazeptionsvorrichtung 1 gezeigt, deren Haltevorrichtung 10 zwei Abstandselemente 19 aufweist. Die Abstandselemente 19 liegen in einander gegenüberliegenden Stützbereichen 20 an Oberflächenabschnitten des Körpers des Säugetiers an. Die Abstandselemente 19 sind mit den ihnen zugeordneten Elektroden 2,3 und Haltevorrichtungen 10 derart in mechanische Wirkverbindung gebracht, dass eine durch die Haltevorrichtung 10 ausgeübte Haltekraft über die Abstandselemente 19 den Stützbereich 20 eingeleitet wird. Bei der dargestellten Kontrazeptionsvorrichtung 1 sind die Stützbereiche 20 nicht dem Körperabschnitt zugeordnet, in dem Hoden 21 oder Nebenhoden 8 des Säugetiers angeordnet ist. Die Elektroden 2,3 mit ihren zugehörigen Abstandselementen 19 sind auf einer einem Gelenk 22 der Kontrazeptionsvorrichtung 1 gegenüberliegenden Seite der Kontrazeptionsvorrichtung 1 angeordnet. Das Gelenk 22 weist einen nicht dargestellten Federmechanismus auf, durch den die Haltekraft ausgeübt wird. Die Elektroden 2,3 mit ihren zugehörigen Abstandselementen 19 sind durch das Gelenk 22 über die Haltevorrichtung 10 mit der Haltekraft beaufschlagt, so dass die Abstandselemente 19 in dem Stützbereich 20 gegen den Hodensack 7 gedrückt werden.

In dem Gelenk 22 wird durch eine Schenkelfeder (nicht dargestellt) bevorzugt ein Drehmoment zwischen 0,06 Nm und 0,16 Nm erzeugt, welches zwischen den Abstandselementen 19 eine Kraft zwischen 1,5 N und 4 N erzeugt. Dadurch wird ein korrekter Anpressdruck der Abstandselemente gewährleistet und die Elektroden richten sich automatisch aus. Eine manuelle Nachjustierung ist nicht notwendig. Der korrekte Sitz der Elektroden kann durch ein manuelles Ertasten des Samenleiters erfolgen. Der Samenleiter muss zwischen den Elektroden 2, 3 austreten.

Durch die Haltevorrichtung 10 ist der Hoden 21 mit dem zugehörigen Nebenhoden 8 von derjenigen Körperseite her umgriffen, der der Hoden 21 zugeordnet ist. Die in Figur 4 dargestellte Kontrazeptionsvorrichtung 1 ist am linken Hoden 21 des Säugetiers angeordnet. Der Hoden 21 ist mitsamt des ihm zugeordneten Nebenhodens 8 um 90° in Richtung einer nicht dargestellten Körpermitte des Säugetiers gedreht.

Zwischen den Elektroden 2,3 und der Hodensackhaut 12 ist jeweils ein Spalt 23 ausgebildet, der mit einem Elektrodengel 24 gefüllt ist.

Figur 6 zeigt eine Schnittansicht einer weiteren Ausführungsform der erfindungsgemäßen Kontrazeptionsvorrichtung 1 mit einer Haltevorrichtung 10 und zwei Abstandselementen 19, die an jeweils einer der Elektroden 2 und 3 befestigt sind, sodass sie in einander gegenüberliegenden Stützbereichen 20 an Oberflächenabschnitten des Körpers des Säugetiers anliegen.

Die Elektroden 2 und 3 sind mit einer Haltevorrichtung 10 derart verbunden, dass die dem zu erzeugenden elektrischen Feld abgewandte Elektrodenseite frei liegt und somit ein Temperaturausgleich durch die Umgebungsluft erfolgen kann. Die Abstandselemente 19 sind feldseitig mit der Elektrode 2, 3 verbunden und ragen etwas über den äußeren Rand der Elektrode 2, 3 und der Haltevorrichtung 10 heraus. Damit wird der Kontakt der Kontrazeptionsvorrichtung 1 mit dem Hoden benachbarten Körperregionen reduziert. Durch die Abstandselemente 19 wird gewährleistet, dass kein Druck beziehungsweise nur ein geringer Druck auf die druckempfindlichen Nebenhoden 8 ausgeübt wird. Die Abstandselemente 19 stützen sich auf der druckunempfindlichen Hodensackhaut ab. Dadurch entsteht im gesamten Anwendungsbereich kein Druckschmerz. Der zwischen den Elektroden 2, 3 und der Hodensackhaut 12 gebildete Spalt 23 ist wie in Fig. 5 mit einem Elektrodengel 24 gefüllt.

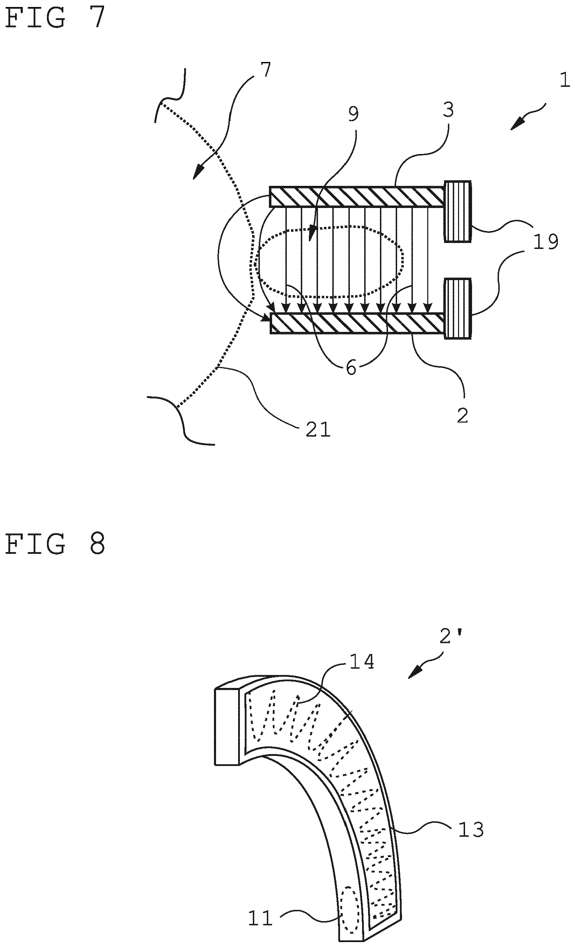

Figur 7 zeigt eine schematische Darstellung des elektrischen Felds, das zwischen der ersten Elektrode 2 und der zweiten Elektrode 3 einer erfindungsgemäßen Kontrazeptionsvorrichtung 1 wirkt. Durch die parallele Anordnung der Elektroden 2 und 3 zueinander entsteht bei Anlegen von Wechselspannung ein durch die parallelen Feldlinien 6 dargestelltes gleichmäßiges elektrisches Feld, das für eine relativ gleichmäßige Erwärmung des in dem elektrischen Feld angeordneten Nebenhodens 9 sorgt. Die elektrische Feldstärke nimmt außerhalb der Elektrodenanordnung mit zunehmendem Abstand zu den Elektroden sehr stark ab, sodass eine Erwärmung des benachbarten Gewebes, insbesondere von an den Nebenhoden 9 angrenzenden Bereichen des Hodens 21, räumlich begrenzt und soweit reduziert ist, dass kein negativer Effekt auf die Hodenfunktionen auftritt. Bevorzugt weisen die Elektroden 2 und 3 eine hohe Wärmeleitfähigkeit auf, sodass es zu einem Ausgleich möglicherweise auftretender Temperaturunterschiede innerhalb des Bereiches zwischen den Elektroden kommt.

In Figur 8 ist eine schematisch dargestellte, perspektivische Ansicht der ersten Elektrode 2' der in Figur 2 gezeigten Kontrazeptionsvorrichtung 1' gezeigt. Die Elektrode 2' weist ein als elektrisches Heizelement 14 ausgebildetes Temperierungselement 13 auf. Der Temperatursensor 11 ist in die Elektrode 2' eingebracht.

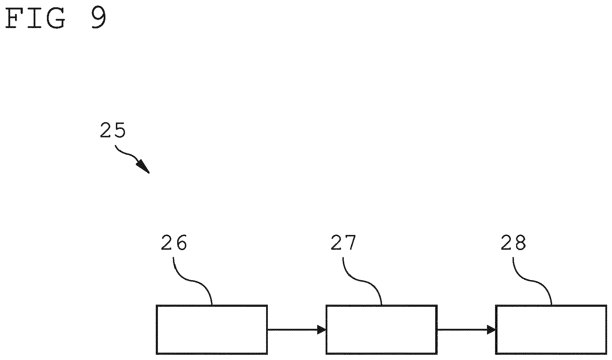

In Figur 9 ist schematisch ein Kontrazeptionsverfahren 25 zur Reduzierung der Qualität von Spermien eines Säugetiers durch ein lokales Erwärmen zumindest eines Nebenhodens des Säugetiers gezeigt, das mittels der zuvor beschriebenen Kontrazeptionsvorrichtungen 1, 1' durchführbar. Bei dem dargestellten Kontrazeptionsverfahren 25 wird zunächst in einem Vorbereitungsschritt 26 ein Elektrodengel auf die Elektroden aufgetragen.

In einem auf den Vorbereitungsschritt folgenden Positionierungsschritt 27 wird eine Kontrazeptionsvorrichtung an dem Körperabschnitt positioniert, wobei Elektroden der Kontrazeptionsvorrichtung derart positioniert werden, dass ein Kontakt zwischen den Elektroden und der Körperoberfläche hergestellt wird. Durch das zuvor in dem Vorbereitungsschritt 26 auf die Elektroden aufgetragene Elektrodengel ist die thermische und elektrische Leitfähigkeit zwischen den Elektroden und der Körperoberfläche verbessert. Durch das Ertasten des Austritts des Samenleiters zwischen den Elektroden 2, 3 kann der korrekte Sitz der Elektroden überprüft werden.

In einem auf den Positionierungsschritt 27 folgenden Erwärmungsschritt 28 wird durch die Kontrazeptionsvorrichtung eine HF-Wechselspannung erzeugt und ein durch die HF-Wechselspannung induzierter elektrischer Strom in den Körperabschnitt eingeleitet, wodurch der zwischen den Elektroden 2, 3 angeordnete Teil des Nebenhodens erwärmt wird und die Qualität von in dem Teil des Nebenhodens enthaltenen Spermien derart einschränkt wird, dass die Fertilität des Säugetiers vorübergehend eingeschränkt ist.

### BEZUGSZEICHENLISTE

- 1.: Kontrazeptionsvorrichtung
- 2.: erste Elektrode
- 3.: zweite Elektrode
- 4.: HF-Generator
- 5.: Elektrodenkabel
- 6.: Feldlinie
- 7.: Hodensack
- 8.: Nebenhoden
- 9.: Gewebe
- 10.: Haltevorrichtung
- 11.: Temperatursensor
- 12.: Hodensackhaut
- 13.: Temperierungselement
- 14.: elektrisches Heizelement
- 15.: Fluidleitung
- 16.: Schlauchsystem
- 17.: Regelungseinrichtung
- 18.: Datenverarbeitungseinrichtung
- 19.: Abstandselement
- 20.: Stützbereich
- 21.: Hoden
- 22.: Gelenk
- 23.: Spalt
- 24.: Elektrodengel
- 25.: Kontrazeptionsverfahren
- 26.: Vorbereitungsschritt
- 27.: Positionierungsschritt
- 28.: Erwärmungsschritt

## Patentansprüche

1. Kontrazeptionsvorrichtung (1) zur Reduzierung einer Qualität von Spermien eines Säugetiers durch ein lokales Erwärmen zumindest eines Nebenhodens (8) des Säugetiers, wobei die Kontrazeptionsvorrichtung (1) eine Elektrodenanordnung mit zumindest einer ersten Elektrode (2, 2') und einer parallel zu der ersten Elektrode (2, 2') ausrichtbaren zweiten Elektrode (3, 3'), und einen HF-Generator (4) zur Erzeugung einer hochfrequenten Wechselspannung aufweist, wobei die Elektroden (2, 2' ,3, 3') derart mit dem HF-Generator (4) in elektrische Wirkverbindung bringbar sind, dass die hochfrequente Wechselspannung zwischen den beiden Elektroden (2, 2',3, 3') anlegbar ist, wobei die Elektroden (2, 2', 3, 3') derart an eine Anatomie des Säugetiers angepasst und/ oder anpassbar sind, dass ein durch die HF-Spannung induzierter elektrischer Strom in zumindest dem Körperabschnitt zumindest einer der Nebenhoden (8) des Säugetiers zumindest abschnittsweise angeordnet ist und wobei durch den elektrischen Strom der Nebenhoden (8) zumindest abschnittsweise erwärmbar ist, sodass die Qualität von in dem Nebenhoden (8) enthaltenen Spermien derart einschränkbar ist, dass die Fertilität des Säugetiers vorübergehend eingeschränkt ist, wobei die Kontrazeptionsvorrichtung (1) zumindest eine Haltevorrichtung (10) aufweist, wobei die Elektrodenanordnung derart an der Haltevorrichtung (10) festlegbar ist, dass die erste Elektrode (2, 2') und die zweite Elektrode (3, 3') einander zugewandt und parallel zueinander ausgerichtet sind,
**dadurch gekennzeichnet, dass** die Kontrazeptionsvorrichtung (1) ein Abstandselement (19) aufweist, wobei das Abstandselement (19) dazu bestimmt ist, in einem Stützbereich an einem Oberflächenabschnitt des Körpers des Säugetiers anzuliegen, wobei das Abstandselement (19), die Elektrodenanordnung und die Haltevorrichtung (10) derart miteinander in mechanischer Wirkverbindung sind, dass eine durch die Haltevorrichtung (10) ausgeübte Haltekraft über das Abstandselement (19) zumindest teilweise in dem Stützbereich in den Körper des Säugetiers einleitbar ist.

2. Kontrazeptionsvorrichtung (1) nach Anspruch 1, **dadurch gekennzeichnet, dass** die beiden Elektroden (2, 2`,3, 3') planare Elektroden sind.

3. Kontrazeptionsvorrichtung (1) nach einem der Ansprüche 1 oder 2, **dadurch gekennzeichnet, dass** die beiden Elektroden (2, 2`,3, 3') ein Material mit einer hohen Wärmeleitfähigkeit aufweisen.

4. Kontrazeptionsvorrichtung (1) nach Anspruch 3, **dadurch gekennzeichnet, dass** die beiden Elektroden (2, 2`,3, 3') ein Material aufweisen, das ein Produkt aus Wärmeleitfähigkeit und Dicke von mindestens 3,8 W·K⁻¹ aufweist.

5. Kontrazeptionsvorrichtung (1) nach Anspruch 4, **dadurch gekennzeichnet, dass** die Haltevorrichtung (10) ein Gelenk (22) aufweist, welches einen Federmechanismus aufweist, durch den zwischen den Abstandselementen (19) eine Kraft zwischen 1,5 N und 4 N erzeugbar ist.

6. Kontrazeptionsvorrichtung (1) nach einem der Ansprüche 1 bis 5, **dadurch gekennzeichnet, dass** die Kontrazeptionsvorrichtung (1) zumindest einen Temperatursensor (11) aufweist, wobei mittels des Temperatursensors (11) eine Oberflächentemperatur eines dem Körperabschnitt zuordenbaren Körperoberflächenbereichs bestimmbar ist.

7. Kontrazeptionsvorrichtung (1) nach Anspruch 6, **dadurch gekennzeichnet, dass** jeweils zumindest ein Temperatursensor (11) an der ersten Elektrode (2, 2') und an der zweiten Elektrode (3, 3') angeordnet sind.

8. Kontrazeptionsvorrichtung (1) nach einem der Ansprüche 1 bis 7, **dadurch gekennzeichnet, dass** die Kontrazeptionsvorrichtung (1) ein Temperierungselement (13) aufweist, wobei das Temperierungselement (13) bei bestimmungsgemäßer Verwendung der Kontrazeptionsvorrichtung (1) mit dem Körperabschnitt in thermische Wirkverbindung bringbar ist und wobei mittels des Temperierungselements (13) thermische Energie in den Körperabschnitt einleitbar und/ oder aus dem Körperabschnitt abführbar ist.

9. Kontrazeptionsvorrichtung (1) nach einem der Ansprüche 1 bis 8, **dadurch gekennzeichnet, dass** die Kontrazeptionsvorrichtung (1) eine Regelungseinrichtung (17) aufweist, wobei die Regelungseinrichtung (17) derart mit dem HF-Generator (4) in Wirkverbindung gebracht/ bringbar ist, dass mittels der Regelungseinrichtung (17) zumindest die durch den HF-Generator (4) erzeugte Wechselspannung regelbar ist.

10. Kontrazeptionsvorrichtung (1) nach Anspruch 9, **dadurch gekennzeichnet, dass** die Regelungseinrichtung (17) eine Datenverarbeitungseinrichtung (18) aufweist, wobei die Datenverarbeitungseinrichtung (18) signal- und/ oder datenleitend mit dem Temperatursensor (11) verbunden/ verbindbar ist und wobei mittels der Datenverarbeitungseinrichtung (18) aufgrund von Temperatursensordaten und anhand einer in der Datenverarbeitungseinrichtung (18) hinterlegten Regel eine Nebenhodentemperatur bestimmbar ist.

11. Kontrazeptionsvorrichtung (1) nach einem der Ansprüche 9 oder 10, **dadurch gekennzeichnet, dass** eine Regelung der Wechselspannung durch die Regelungseinrichtung (17) zumindest teilweise auf der Grundlage von Temperatursensordaten des Temperatursensors (11) erfolgt.

## Claims

1. Contraceptive device (1) for reducing the sperm quality of a mammal by locally heating at least one epididymis (8) of the mammal, wherein the contraceptive device (1) comprises an electrode arrangement with at least one first electrode (2, 2') and a second electrode (3, 3') which can be aligned parallel to the first electrode (2, 2'), and a high-frequency generator (4) for generating a high-frequency alternating voltage, wherein the electrodes (2, 2', 3, 3') can be brought into electrically active connection with the high-frequency generator (4) in such a way that the high-frequency alternating voltage can be applied between the two electrodes (2, 2', 3, 3'), wherein the electrodes (2, 2', 3, 3') are adapted and/or adaptable to a mammalian anatomy such that an electric current induced by the HF voltage is arranged at least in sections in at least the body section of at least one of the mammalian epididymides (8) and the epididymis (8) can be heated at least in sections by the electric current, so that the quality of the sperm contained in the epididymis (8) can be limited in such a way that the fertility of the mammal is temporarily restricted, wherein the contraceptive device (1) comprises at least one holding device (10), the electrode arrangement (30) can be attached to the holding device (10) in such a way that the first electrode (2, 2') and the second electrode (3, 3') face one another and are aligned parallel to one another,
**characterized in that** the contraceptive device (1) comprises a spacer element (19), wherein the spacer element (19) is intended to bear against a surface portion of the mammal's body in a support region, the spacer element (19), the electrode arrangement and the holding device (10) are in mechanical operative connection with one another in such a way that a holding force exerted by the holding device (10) can be introduced into the body of the mammal via the spacer element (19) at least partially in the support region.

2. Contraceptive device (1) according to claim 1,
**characterized in that** the two electrodes (2, 2', 3, 3') are planar electrodes.

3. Contraceptive device (1) according to any one of claims 1 or 2, **characterized in that** the two electrodes (2, 2', 3, 3') comprise a material with high thermal conductivity.

4. Contraceptive device (1) according to claim 3, **characterized in that** the two electrodes (2, 2', 3, 3') have a material which has a product of thermal conductivity and thickness of at least 3.8 W·K⁻¹.

5. Contraceptive device (1) according to claim 4, **characterized in that** the holding device (10) comprises a joint (22) which comprises a spring mechanism by means of which a force of between 1.5 N and 4 N can be generated between the spacer elements (19).

6. Contraceptive device (1) according to any one of claims 1 to 5, **characterized in that** the contraceptive device (1) comprises at least one temperature sensor (11), wherein a surface temperature of a body surface region which can be assigned to the body section is determinable by means of the temperature sensor (11).

7. Contraceptive device (1) according to claim 6,
**characterized in that** at least one temperature sensor (11) is arranged on the first electrode (2, 2') and on the second electrode (3, 3'), respectively.

8. Contraceptive device (1) according to any one of claims 1 to 7, **characterized in that** the contraceptive device (1) comprises a thermoregulation element (13), wherein the thermoregulation element (13) is capable of being brought into thermal operative connection with the body portion when the contraceptive device (1) is used as intended, and wherein thermal energy can be introduced into the body portion and/or discharged from the body portion by means of the thermoregulation element (13).

9. Contraceptive device (1) according to any one of claims 1 to 8, **characterized in that** the contraceptive device (1) comprises a control unit (17), wherein the control unit (17) is operatively connected to the HF generator (4) in such a way that at least the AC voltage generated by the HF generator (4) can be controlled by means of the control unit (17).

10. Contraceptive device (1) according to claim 9, **characterized in that** the control unit (17) comprises a data processing unit (18), wherein the data processing unit (18) is connected/connectable to the temperature sensor (11) via a signal connection and/or data connection, and wherein an epididymal temperature is determinable by means of the data processing unit (18) on the basis of data from the temperature sensor and with the aid of a rule stored in the data processing unit (18).

11. Contraceptive device (1) according to any one of claims 9 or 10, **characterized in that** regulation of the AC voltage by the regulating device (17) is effected at least in part on the basis of temperature sensor data from the temperature sensor (11) .

## Revendications

1. Dispositif contraceptif (1) pour réduire la qualité des spermatozoïdes d'un mammifère en chauffant localement au moins un épididyme (8) du mammifère, le dispositif contraceptif (1) présentant un agencement d'électrodes avec au moins une première électrode (2, 2') et une deuxième électrode (3, 3') pouvant être orientée parallèlement à la première électrode (2, 2'), et un générateur HF (4) pour produire une tension alternative à haute fréquence, les électrodes (2, 2', 3, 3') pouvant être amenées en liaison active électrique avec le générateur HF (4) de telle sorte que la tension alternative à haute fréquence peut être appliquée entre les deux électrodes (2, 2', 3, 3'), les électrodes (2, 2', 3, 3') étant adaptées et/ou adaptables à une anatomie du mammifère de telle sorte qu'un courant électrique induit par la tension HF est agencé au moins par sections dans au moins la section de corps d'au moins l'une des épididymes (8) du mammifère et l'épididyme (8) pouvant être chauffé au moins par sections par le courant électrique, de telle sorte que la qualité des spermatozoïdes contenus dans l'épididyme (8) peut être limitée de telle sorte que la fertilité du mammifère est temporairement limitée, le dispositif contraceptif (1) présentant au moins un dispositif de maintien (10), l'agencement d'électrodes (30) pouvant être fixé sur le dispositif de maintien (10) de telle sorte que la première électrode (2, 2') et la deuxième électrode (3, 3') sont tournées l'une vers l'autre et orientées parallèlement l'une à l'autre,
**caractérisé en ce que** le dispositif contraceptif (1) présente un élément d'espacement (19), l'élément d'espacement (19) étant destiné à s'appliquer contre une section de surface du corps du mammifère dans une zone d'appui, l'élément d'espacement (19), l'agencement d'électrodes et le dispositif de maintien (10) étant en liaison mécanique active les uns avec les autres de telle sorte qu'une force de maintien exercée par le dispositif de maintien (10) peut être introduite dans le corps du mammifère par l'intermédiaire de l'élément d'espacement (19) au moins partiellement dans la zone d'appui.

2. Dispositif contraceptif (1) selon la revendication 1, **caractérisé en ce que** les deux électrodes (2, 2', 3, 3') sont des électrodes planes.

3. Dispositif contraceptif (1) selon l'une quelconque des revendications 1 ou 2, **caractérisé en ce que** les deux électrodes (2, 2', 3, 3') présentent un matériau ayant une conductivité thermique élevée.

4. Dispositif contraceptif (1) selon la revendication 3, **caractérisé en ce que** les deux électrodes (2, 2', 3, 3') présentent un matériau qui présente un produit de la conductivité thermique et de l'épaisseur d'au moins 3,8 W·K⁻¹.

5. Dispositif contraceptif (1) selon la revendication 4, **caractérisé en ce que** le dispositif de maintien (10) présente une articulation (22) qui présente un mécanisme à ressort par lequel une force comprise entre 1,5 N et 4 N peut être produite entre les éléments d'espacement (19).

6. Dispositif contraceptif (1) selon l'une quelconque des revendications 1 à 5, **caractérisé en ce que** le dispositif contraceptif (1) présente au moins un capteur de température (11), une température de surface d'une zone de surface de corps pouvant être associée à la section de corps pouvant être déterminée au moyen du capteur de température (11).

7. Dispositif contraceptif (1) selon la revendication 6, **caractérisé en ce qu'**au moins un capteur de température (11) est agencé respectivement sur la première électrode (2, 2') et sur la deuxième électrode (3, 3').

8. Dispositif contraceptif (1) selon l'une quelconque des revendications 1 à 7, **caractérisé en ce que** le dispositif contraceptif (1) présente un élément de thermorégulation (13), l'élément de thermorégulation (13) pouvant être amené en liaison active thermique avec la section de corps lors de l'utilisation conforme du dispositif contraceptif (1) et de l'énergie thermique pouvant être introduite dans la section de corps et/ou évacuée de la section de corps au moyen de l'élément de thermorégulation (13).

9. Dispositif contraceptif (1) selon l'une quelconque des revendications 1 à 8, **caractérisé en ce que** le dispositif contraceptif (1) présente un appareil de régulation (17), l'appareil de régulation (17) étant mis/pouvant être mis en liaison active avec le générateur HF (4) de telle sorte qu'au moyen de l'appareil de régulation (17), au moins la tension alternative produite par le générateur HF (4) peut être régulée.

10. Dispositif contraceptif (1) selon la revendication 9, **caractérisé en ce que** l'appareil de régulation (17) présente un appareil de traitement de données (18), l'appareil de traitement de données (18) étant relié/pouvant être relié au capteur de température (11) par une liaison de signaux et/ou de données et une température de l'épididyme pouvant être déterminée au moyen de l'appareil de traitement de données (18) sur la base de données du capteur de température et à l'aide d'une règle mémorisée dans l'appareil de traitement de données (18).

11. Dispositif contraceptif (1) selon l'une quelconque des revendications 9 ou 10, **caractérisé en ce qu'**une régulation de la tension alternative par l'appareil de régulation (17) est effectuée au moins en partie sur la base de données de capteur de température du capteur de température (11).
